# EUROPEAN PATENT APPLICATION

(11) **EP 2 633 855 A1**
(43) Date of publication of application: **04.09.2013**
(21) Application number: 12157707.6
(22) Date of filing: 01.03.2012
(51) Int. Cl.: A61K 31/18, A61K 31/336, A61K 39/002, A61K 39/39, A61P 33/02

(54) **Protease inhibitors for treating Trichomonas gallinae infections**

(71) Applicant: Veterinärmedizinische Universität Wien, 1210 Vienna (AT)
(72) Inventor: Hess, Michael, 3400 Klosterneuberg (AT); Bilic, Ivana, 1210 Vienna (AT); Abdel-Fattah Abdel-Mageed Amin, Aziza, 1210 Vienna (AT)
(74) Representative: Sonn & Partner Patentanwälte

(57) **Abstract**

Disclosed is the use of protease inhibitors for use in the treatment of a Trichomonas gallinae infection of an animal, selected from the orders Galliformes, Passeriformes, Psittaciformes, Columbiformes and Falconiformes.

SEQUENCE LISTING

<110>  VETERINÄRMEDIZINISCHE UNIVERSITÄT WIEN

<120>  Use of protease inhibitors

<130>  R 61289

<160>  6

<170>  PatentIn version 3.5

<210>  1
<211>  12
<212>  PRT
<213>  Trichomonas gallinae

<400>  1

Phe Ser Tyr Ile Ala Asp Tyr Pro Tyr Thr Ala Arg
1               5                   10


<210>  2
<211>  12
<212>  PRT
<213>  Trichomonas gallinae

<400>  2

Phe Ser Tyr Leu Ala Asp Tyr Pro Tyr Thr Ala Arg
1               5                   10


<210>  3
<211>  10
<212>  PRT
<213>  Trichomonas gallinae

<400>  3

Asn Ser Trp Gly Ala Ser Trp Gly Glu Lys
1               5                   10


<210>  4
<211>  6
<212>  PRT
<213>  Trichomonas gallinae

<400>  4

Asn Tyr Trp Ile Val Arg
1               5


<210>  5
<211>  6
<212>  PRT
<213>  Trichomonas gallinae

<400>  5

Asn Tyr Trp Leu Val Arg
1               5

<210>  6
<211>  14
<212>  PRT
<213>  Trichomonas gallinae

<400>  6

Val Asn Val Val Glu Gly Asp Glu Ala Asp Leu Ala Thr Lys
1               5                   10

## Description

The present invention relates to the use of protease inhibitors, especially cysteine protease inhibitors.

Trichomonas gallinae is a flagellated protozoan parasite which infects a variety of birds all over the world. Avian trichomonosis, caused by T. gallinae, has been reported from several continents as a major disease for numerous avian species of the orders Columbiformes, Falconiformes and Psittacifomes. They may infect other avian species as well, like Galliformes and Passeriformers. The domestic pigeon (Columba livia) is the primary host of this flagellate which has been considered responsible for the worldwide spread of T. gallinae. Furthermore, serious losses among wild birds, in particular wild finches, due to T. gallinae were reported recently. T. gallinae colonizes mainly in the upper digestive tract of the birds, where, it can cause granulomatous lesions that occlude the oesophagus lumen, leading to the death of birds as a result of severe starvation. The parasite produces a variety of pathological changes depending on the virulence of the strain and the species of bird infected. Earlier studies with T. gallinae have demonstrated a wide spectrum of virulence among different T. gallinae strains. Accordingly, some T. gallinae strains do not cause clinical signs and can induce certain immunity against a highly virulent one. Virulent strains are able to produce a systemic infection in its host and affect mainly the liver and lung beside the oropharynx. Pathological changes following experimental infection of a pigeon with a virulent strain of T. gallinae were described in detail in the prior art.

Applying molecular technologies genetic differences between T. gallinae strains could be demonstrated in different studies, for example it could be shown that T. gallinae clones 5895-C1/06 and 8855-C3/06 are members of a particular clade (T. gallinae like isolates) belonging to two different subgroups. In contrast clone 231-C1/07 was grouped in a different clade (Trichomonas tenax like isolates), based on phylogenetic analyses of the two ribosomal RNA internal transcribed spacers (ITS1 and ITS2) and the 5.8S rRNA gene.

In order to assess the virulence of T. gallinae in vivo a suitable system is needed premising a simple working protocol and the protection of animals. Embryonated chicken eggs have been one of the most common substrate for isolation, propagation and characterization of different avian viruses as well as for the production of viral vaccines. Additionally, embryonated eggs have been used to investigate the virulence of various bacteria and fungi.

Recently, peptidases are described as synonyms for proteases, proteinases and peptide hydrolases which are the best known enzymes which catabolize proteins and polypeptides through cleavage of peptide bonds. Cysteine peptidases have been reported as essential factors in the biology and the pathogenesis of various parasitic diseases. In this context it was reported that cysteine peptidases are involved in degradation of extracellular matrix components such as fibronectin and laminin. Previously, it was demonstrated that liver abscesses produced by Entamoeba histolytica in severe combined immunodeficient (SCID) mice were greatly reduced by preincubation of the trophozoites with the cysteine peptidase inhibitor namely L-3-carboxyl-2,3-trans-epoxysuccinyl-leucylamido (4-guanidino) butane (E-64). Moreover, application of a specific inhibitor to major Trypanosoma cruzi cysteine peptidases (cruzain) was able to protect the infected mice from lethal infection.

Recently, cathepsin L-like cysteine peptidases were identified as secreted virulence products in a cell-free filtrate of axenic T. gallinae clonal culture. Additionally, it could also be shown that certain peptidase inhibitors are capable to reduce the cytopathogenic effects of T. gallinae in a permanent chicken liver (LMH) cell line. However, evidence that peptidase inhibitors could significantly decrease the protozoal peptidases of T. gallinae activity in vivo is still lacking.

It is therefore an object of the present invention to provide suitable means for combatting infections with T. gallinae in birds.

Therefore the present invention provides protease inhibitors for use in the treatment of a Trichomonas gallinae infection of an animal, selected from the orders Galliformes, Passeriformes, Psittaciformes, Columbiformes and Falconiformes.

With the present invention it is demonstrated that the progression of avian trichomonosis is linked with the presence of cysteine peptidases which can be neutralized by co-application of peptidase inhibitors without toxicity to the host. Such peptidase inhibitors had no direct cytotoxic effect on the viability of the trichomonads. This indicates that the reduction of pathogenicity of T. gallinae in the presence of peptidase inhibitors is due to the inhibition of cysteine peptidase activity rather than a direct effect on the trichomonad cells. The present investigations also show the importance of cysteine peptidases as drug targets as a strategy for chemotherapy.

The present invention is based on the surprising fact that the protease inhibitors are successful in preventing or reducing the pathogenic effects of T. gallinae after oral administration to the animals. Although proteases have been suggested for combatting parasitic diseases for more than 20 years (e.g. Rosenthal et al., J. Clin. Invest. 82 (1988), 1560-1566), no successful application of this concept has yet made its way to applied medicine, neither in humans nor in animals. This might also be due to the different nature and protease strategy such pathogens have with respect to their host.

The fact that the strategy of the present invention can be successfully applied for combatting T. gallinae caused pathogenicity stands in contrast to recently published results that the haemolytic activity of T. gallinae does not correspond with clinical virulence (Gerhold et al., Vet. Parasitol. 160 (2009), 221-224). The successful administration of protease inhibitors according to the present invention shows that those results do not necessarily exclude combatting clinical virulence of T. gallinae, although the results according to the present invention could not have been predicted on the basis of e.g. Gerhold et al.. For example, it was also surprising that T. gallinae, in contrast to the closely related Tetratrichomonas gallinarum (Malik et al., PLoS ONE 6(2011): e20774) indeed induces distinctive cytopathogenic effects in tissue cultures.

Preferably, the protease inhibitors according to the present invention are protease inhibitors selected from cysteine protease inhibitors and serine protease inhibitors, especially cysteine protease inhibitors.

Proteases are enzymes that degrade polypeptides.

Cysteine proteases have a common catalytic mechanism that involves a nucleophilic cysteine thiol in a catalytic dyad. The first step is deprotonation of a thiol in the enzyme's active site by an adjacent amino acid with a basic side chain, usually a histidine residue. The next step is nucleophilic attack by the deprotonated cysteine's anionic sulfur on the substrate carbonyl carbon. In this step, a fragment of the substrate is released with an amine terminus, the histidine residue in the protease is restored to its deprotonated form, and a thioester intermediate linking the new carboxy-terminus of the substrate to the cysteine thiol is formed. Therefore they are also sometimes referred to as thiol proteases. The thioester bond is subsequently hydrolysed to generate a carboxylic acid moiety on the remaining substrate fragment, while regenerating the free enzyme.

Serine proteases are enzymes that cleave peptide bonds in proteins, in which serine serves as the nucleophilic amino acid at the active site. Serine proteases fall into two broad categories based on their structure: chymotrypsin-like (trypsin-like) or subtilisin-like. The main player in the catalytic mechanism in the serine proteases is the catalytic triad. The triad is located in the active site of the enzyme, where catalysis occurs, and is preserved in all serine protease enzymes. The triad is a coordinated structure consisting of three essential amino acids: histidine, serine ("serine protease") and aspartic acid. Located very near one another near the heart of the enzyme, these three key amino acids each play an essential role in the cleaving ability of the proteases. In the event of catalysis, an ordered mechanism occurs in which several intermediates are generated. The catalysis of the peptide cleavage can be seen as a ping-pong catalysis, in which a substrate binds (in this case, the polypeptide being cleaved), a product is released (the N-terminus "half" of the peptide), another substrate binds (in this case, water), and another product is released (the C-terminus "half" of the peptide).

Inhibitors to proteases are known to the person skilled in the art. In fact, numerous protease inhibitors with specificity to e.g. serine proteases or cysteine proteases are available.

According to a preferred embodiment of the present invention, the protease inhibitors are cysteine protease inhibitors selected from the group consisting of E-64 (trans-Epoxysuccinyl-L-leucylamido(4-guanidino)butane, L-trans-3-Carboxyoxiran-2-carbonyl-L-leucylagmatine, N-(trans-Epoxysuccinyl)-L-leucine 4-guanidinobutylamide), TLCK (tosyl-L-lysine chloromethyl ketone), TPCK (tosyl-L-phenylalanine chloromethyl ketone), or mixtures thereof.

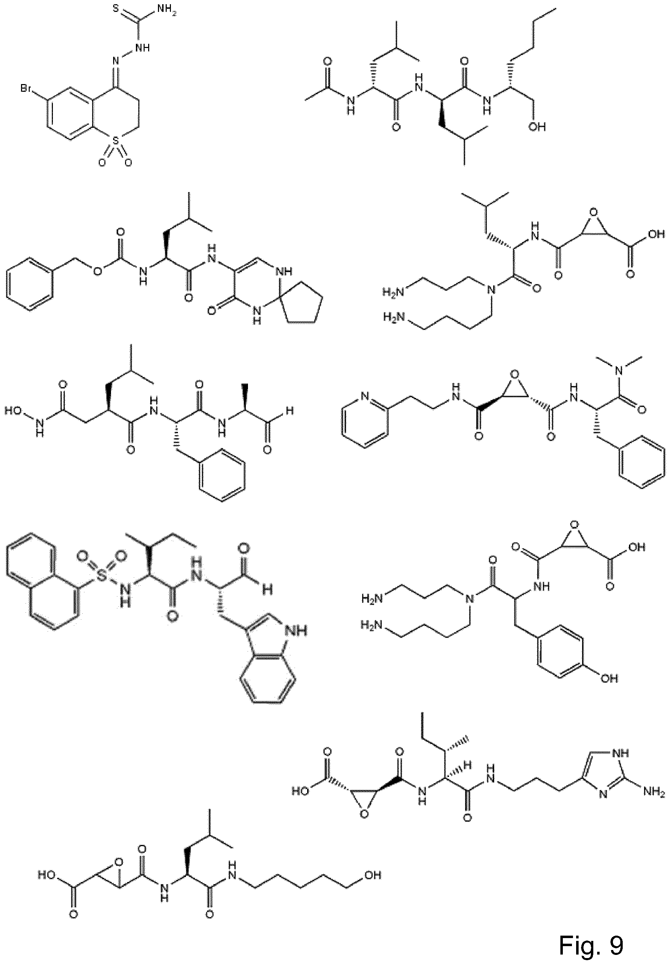

A group of preferred cysteine protease inhibitors is the group of cathepsin L inhibitors, such as thiochromanone thiosemicarbazone analogs 6-bromo-TST, acetyl-leu-leu-norleucinol, NPI-8343, NPI-8344, NPI-2349, NPI-2019, NPI-3485 and NPI-3469, epoxysuccinyl acid derivatives containing aziridine-2,3-dicarboxylic acid as the electrophilic alpha-amino acid, inhibitors as disclosed in JP-00987265 A, FEBS Lett. 458 (1999), 6-10, EP 0 0611 756 A, and Antibiotics 51 (1998), 629-634; 3-(N-(1-(N-(4-aminobutyl) N-(3-aminopropyl) carbamoyl) 2-(4-hydroxyphenyl) ethyl) carbamoyl) oxirane 2-carboxylic acid, WF-14685A and WF-14865B. Examples are also given in Fig. 9.

Cysteine protease inhibitors are also (among many other documents) disclosed in WO 99/53039 A1, WO 2007/012180 A1, WO 2009/067797 A1, WO 2010/054042 A2, WO 94/06280 A1, WO 95/23229 A1 and Rosenthal et al., J.Clin. Invest 82 (1988), 1560-1566). Cysteine protease inhibitors, including their functionality and mechanistic properties are also reviewed by Otto et al. (Chem. Rev. 97 (1997), 133-171, especially 147-164), e.g. peptidylaldehydes, such as leupeptins, chymostatins, antipain, elastinal and β-MAPI (β-microbial alkaline protease inhibitor); peptidyl-semicarbazones, such as Z-Arg-Ile-Phe-Sc, Z-Ile-Phe-Sc, Z-Phe-Gly-Sc and Z-Gly-Phe-Gly-Sc; peptidyl-methyl ketones and - trifluormethyl ketones, such as Z-Phe-Ala-CH₃, Z-Phe-Ala-CF₃, Ac-Phe-Gly-CH₃, Z-Phe-CH₃, Bz-Phe-CHF₂, Bz-Phe-CF₃, Z-Val-Phe-CF₃ and Z-Val-D-Phe-CF₃; peptidyl-α-keto acids, -α-keto esters, -α-keto amides and -diketones, such as Bz-Phe-COOMe, Z-Phe-COOEt, Z-Phe-COCH₃, Z-Val-Phe-COOMe, Z-Val-Phe-COCH₃, Z-Phe-Gly-COOH, Z-Phe-Gly-COOCH₃, Ac-Phe-Gly-COOCH₃, Ac-Phe-Gly-COCH_{3,} Z-Phe-Gly-CONHEt, Z-Phe-Gly-CO-LeuOMe, Z-Phe-Gly-COOn-But, Z-Gly-Phe-Gly-COOn-But, Z-Phe-Gly-CO₂CH₂CO₂Et, Z-Phe-Gly-CO₂(CH₂)₃CO₂Me, 2-Leu-Phe-CONH, Z-Leu-α-aminodimethylacetic acid-CONH₂, Z-Leu-Phe-CONHR, Z-Leu-α-aminodimethylacetic acid-CONHR, Z-Leu-Phe-CONEt₂, Z-Leu-α-aminodimethylacetic acid -CONR₂, Z-Leu-Phe-COOH, Z-Leu-α-aminodimethylacetic acid-COOH, Z-Leu-Phe-COOEt, Z-Leu-α-aminodimethylacetic acid-COOR, Z-Leu-nLeu-COOEt, Z-Leu-Met-COOEt, Z-Leu-α-aminodimethylacetic acid-CO-NHEt-SO₂Et, (CH₃)₂N-CO-Leu-α-aminodimethylacetic acid-CONHEt and morpholino-CO-Leu-α-aminodimethylacetic acid-CONHEte; peptidyl-nitriles, such as Z-NH-CH(CH₂OH)-CN, Ac-NH-CH₂-CN, Bz-NH-CH₂-CN, Ac-Phe-NH-CH₂-CN, CH₃-O-CO-Phe-NH-CH₂-CN, Ac-Phe-NH-CH(i-But)-CN, Gly-NH-CH(CH₂PH)-CN, Ac-NH-CH₂-CSNH₂, Bz-NH-CH2-CSNH₂ and CH3-O-CO-Phe-NHCH₂-CSNH₂; peptidyl-halomethyl ketones, such as TLCK, 2-Phe-Phe-CH₂Cl, Z-Phe-Phe-CH₂F, Z-Phe-Ala-CH₂Cl, 2-Phe-Ala-CH₂F, 2-Leu-Tyr-CH₂F, Z-Ala-Phe-CH₂F, Z-Leu-Leu-Tyr-CH₂F, Z-Tyr-Ala-CH₂F, Z-Leu-Leu-Phe-CH₂Cl, Pro-Phe-Arg-CH₂Cl, Leu-Leu-Phe-CH₂Cl, Leu-Leu-Lys-CH₂Cl, Ala-Phe-Lys-CH₂Cl, Ala-Phe-Lys-CH₂F, Z-Leu-Gly-CH₂Cl, Z-Leu-Gly-CH₂Br, 2-Leu-Ala-CH₂Cl and 2-Leu-Phe-CH₂Cl; peptidyldiazomethanes, such as Z-Phe-Ala-CHN₂, Z-Phe-Thr(OBzl)-CHN₂ Z-Ala-Ala-Pro-CHN₂, 2-Gly-Pro-CHN₂, 2-Lys-CHN₂, 2-Ala-Phe-Ala-CHN₂, Z-Pyrod-Glu-CHN₂, Pyro-CHN₂, Z-Leu-Val-Gly-CHN₂, Z-Phe-Tyr(O-t-But)-CHN₂, Ser(OBzl)-CHN₂, Gly-Phe-CHN₂, Z-Leu-Leu-Tyr-CHN₂ and Z-Tyr(I)-Ala-CHN₂; peptidylacyloxymethyl ketones, such as Z-Phe-Ala-CH₂-O-CO- (2, 6- (CF₃) ₂) -Ph, 2-Phe-Ala-CH₂-O-CO- (2,5- (CF₃) ₂) -Ph, 2-Phe-Ala-CH₂-O-CO-(2,6-Me₂-4-COOMe)-Ph, Z-Phe-Ala-CH₂-O-CO-4-NO₂-Ph, 2-Phe-Ala-CH₂-O-CO-(2,4,6-Me₃)-Ph, Z-Phe-Ala-CH₂-O-CO-CMe₃, Z-Phe-Ala-CH₂-O-C₆F₅, Z-Phe-Lys-CH₂-O-CO-(2,6-(CF₃)₂)-Ph, Z-Phe-Lys-CH₂-O-CO-(2,4_{,}6-Me₃)-Ph, Z-Phe-Ser(OBzl)-CH₂-O-CO-(2,6-(CF₃)₂)-Ph, 2-Phe-Cys(SBzl)-CH₂-O-CO-(2,6-(CF₃)₂)-Ph,2-Leu-Leu-CH₂-O-CO-(2,6-(CF₃)₂)-Ph, Z-Val-Phe-CH₂-O-CO-(2,6-(CF₃)₂)-Ph, Z-Leu-Leu-Phe-CH₂-O-CO-(2,6- (CF₃)₂)-Ph, 2-Asp-CH₂-O-CO-(2,6-Cl₂-Ph), Z-Val-Asp-CH₂-O-CO-(2,6-Cl₂-Ph), 2-Val-Ala-Asp-CH₂-O-CO-(2,6-Cl₂-Ph) and Z-Glu-CH₂-O-CO-(2,6-Cl₂-Ph); peptidyl-methlysulfonium salts, such as (CH₃)₃-S⁺, Z-Phe-CH₂-S⁺- (Me)2, Z-Phe-CH₂-S⁺- (Me) (Bzl), Z-Phe-CH₂-S⁺-(Et)₂ Z-Phe-Ala-CH₂-S⁺-(Me)₂, Z-Phe-Lys-CH₂-S⁺-(Me)₂, Z-Phe-Lys-CH₂-S⁺- (Me) (Bzl) , Z-Lys-CH₂-S⁺-(Me)₂, Ala-Lys-Lys-CH₂-S⁺- (Me) 2, Ala-Lys-Arg-CH₂-S⁺- (Me) ₂, Ala-Arg-Lys-CH₂-S⁺- (Me) ₂, Ala-Arg-Arg-CH₂-S⁺- (Me) ₂, Bz-Phe-Arg-CH₂-S⁺- (Me) ₂ and Z-Leu-Leu-Phe-CH₂-S⁺-(Me)₂; eposuccinyl peptides, such as E-64, E-64 D, Ep 459 (E-64a; HO-Eps-Leu-NH-(CH₂)₄-NH₂), Ep 460 (E-64aZ; HO-Eps-Leu-NH-(CH₂)₄-NH-Z), Ep 459-Ac, Ep 479 (HO-Eps-Leu-NH-(CH₂)₇-NH₂), Ep 174 (HO-Eps-Leu), Eps(Eps=2(S),3(S)-transepoxysuccinate)LeuOBzlb, Ep 47 LL, (E-64c) LD, DL, DD, Ep 420 (Bzl-DL-Eps-Ile-TyrOMe), Ep 429 (E-64b; HO-Eps-Leu-Leu), EpsLeuProOBzl, EtO-EpsLeuProOBzl, i-ButNH-EpsLeuProOBzl, EpsLeuPro, Et-EpsLeuPro, i-ButNH-EpsLeuPro, EpsPheOBzl, EpsArgOBzl, EpsIleOBzl, EpsPheNBzl, EpsLeuNBzl, E-64, E-64c, E-64d, CA-074 (nPr-NH-Eps-Ile-Pro), CA-028 (HO-Eps-Ile-Pro), CA-030 (Et-OEps-Ile-Pro), CA-030-OMe, EtO-Eps-Pro-Pro, EtO-Eps-Thr-Ile, EtO-Eps-Ile-Ala, EtO-Eps-Gly-Pro, HO-Eps-Leu-OEt, HO-Eps-Leu-OBzl, Bzl-O-Eps-Leu-OEt, Ho-Eps-Ile-OBzl, HO-Eps-Phe-OEt, HO-Eps-Orn(Z)-OEt, HO-Eps-Arg(NO₂)-OMe, HO-Eps-Arg-OMe, HO-Eps-Arg and HO-Eps-Lys(Z)-OBzl; unsaturated derivatives using the Michael system, such as DC-11, Fum(Fum=EtOOCCH=CH-CO trans)-Phe-OBzl Fum-Ile-NH-nBu, Fum-Ile-Pro-OBzl, N-ethylmaleimide, N-butylmaleimide, N-hexylmaleimide, N-decylmaleimide, Ac-Phe-NH-CH₂-CH=CH-COOMe, Ac-Phe-NH-CH2-CH=CH, Gly-NH-CH(Bzl)-X (X=CH=CHCOOMe trans), Gly-NH-CH(Bzl)-Y (Y= CH=CHSO₂Me trans), Ac-NH-CH(Bzl)-X, Ac-NH-CH(Bzl)-Y, NH2-CH(Bzl)-X, NH2-CH(Bzl)-Y and Mu(morpholine urea)-Phe-Hphe(homophenylalanine) -VsPh(vinylsulfonyl)benzene CH=CHSO₂Phe); disulfides, azapeptides, azobenzenes, O-acylhydroxamates, lysosomotropic bases, such as ammonium chloride, methylamine, tributylamine, nigericin, gramicidin and chloroquine; calmodulin antagonists, aziridines and thiiranes, 1,3,2-dioxathiolane dioxide derivatives, Ac-Gly-Phe-Nle-OH, Ac-Gly-Phe-Nle-OSO₂-CH₃, aspartyl R-[(1-phenyl-3-(trifluoromethyl)pyrazol-5-yl]-oxy]methyl ketones and aspartyl R-[(diphenylphosphinyl)oxy]-methyl ketones.

Administration amounts are typically dependent on the individual inhibitory capacity of each inhibitor and its toxicity. Preferred administration dosages for the inhibitors are in the mg range, such as 1 to 1000 mg/kg bodyweight/day, preferably 5 to 500 mg/kg bodyweight/day, especially 10 to 100 mg/kg bodyweight/day.

Cysteine protease inhibitors have been specifically effective in the present invention, especially for combatting T. gallinae pathogenicity in preferred avian species. This has been confirmed within the course of the present invention by using chicken embryos, as a disease model. Accordingly, it is preferred to use the protease inhibitors according to the present invention in the treatment of a T. gallinae infection of an animal, selected from a species belonging to the orders Galliformes, Columbiformes, Psittaciformes or Passeriformes, especially Columba livia, Melopsittacus undulates, Serinus canaria forma domestica, or Falco spp..

It could be shown in the course of the present invention that the protease inhibitors according to the present invention can be administered orally to the animal, which makes them specifically suitable for mass application. This was demonstrated in a disease model by applying protease inhibitors via the allantoic cavity to chicken embryos, which took up the parasite and the inhibitors orally.

Preferably, the (cysteine) protease inhibitors according to the present invention (which, as outlined above can be a single inhibitor species or a mixture of two or more different inhibitors), are for use in the oral treatment of a Trichomonas gallinae infection of an animal, especially wherein oral treatment includes administration of a solution of cysteine protease inhibitors or cysteine protease inhibitors in tablet form.

A comfortable route of administration is via drinking water for the birds. In this embodiment, the protease inhibitors according to the present invention are provided in dried form, e.g. as a powder or a lyophilisate, and reconstituted with water or another drinkable aqueous solution before use.

Alternatively, the inhibitors are provided in administration (daily) dosage form, such as capsules, dragees, tablets, etc.. For example, tablets (as well as all other routine dosage formats) can be made from the inhibitors by combining those with appropriate formulation ingredients. The tablet for use in the present invention is composed of a mixture of the (cysteine) protease inhibitors and excipients, usually in powder form, pressed or compacted from a powder into a solid dose. The excipients can include diluents, binders or granulating agents, glidants (flow aids) and lubricants to ensure efficient tabletting; disintegrants to promote tablet break-up in the digestive tract. A polymer coating can be applied to make the tablet smoother and easier to swallow, to control the release rate of the (cysteine) protease inhibitor or to make it more resistant to the environment (extending its shelf life). The present invention also relates to a method for treating a Trichomonas gallinae infection of an animal, selected from the orders Galliformes, Passeriformes, Psittaciformes, Columbiformes and Falconiformes, wherein an effective amount of a protease inhibitor, especially a cysteine protease inhibitor, is administered to an animal.

According to another embodiment, the present invention concerns the use of a protease inhibitor, especially a cysteine protease inhibitor for the manufacture of a medicament for the treatment of a Trichomonas gallinae infection of an animal, selected from the orders Galliformes, Passeriformes, Psittaciformes, Columbiformes and Falconiformes.

Another aspect of the present invention relates to a vaccine against a Trichomonas gallinae infection of an animal, selected from the orders Galliformes, Passeriformes, Psittaciformes, Columbiformes and Falconiformes, comprising a cysteine protease and an adjuvant. Based on the results obtained with the oral administration of the protease inhibitors according to the present invention, also the use of cysteine proteases as active immunogen for the prevention of infections with T. gallinae (or the prevention of the pathogenic effects associated therewith) is an aspect of the present invention. The vaccine containing the cysteine protease can also be provided as a DNA-vaccine comprising a nucleic acid molecule, especially a DNA molecule, encoding the cysteine protease (i.e. containing a cysteine protease gene which can be expressed after delivery of the vaccine to the animal and elicit an immune response to the encoded cysteine protease after expression in the vaccinated animal). Accordingly, the cysteine proteases (or the DNA vaccine) can be combined with any suitable adjuvant which is effective in eliciting an immune response against the cysteine protease (either directly or via the DNA vaccine) in the animal.

"Adjuvant" refers to a compound or mixture that enhances the immune response to an antigen. An adjuvant can serve as a tissue depot that slowly releases the antigen and also as a lymphoid system activator that non-specifically enhances the immune response. Often, a primary challenge with an antigen alone, in the absence of an adjuvant, will fail to elicit a humoral or cellular immune response. Adjuvants include, but are not limited to, complete Freund's adjuvant, incomplete Freund's adjuvant, saponin, mineral gels such as aluminum hydroxide, aluminium sulfate, surface active substances such as lysolecithin, pluronic polyols, polyanions, peptides, oil or hydrocarbon emulsions, keyhole limpet hemocyanins, dinitrophenol, and potentially useful human adjuvants such as BCG (Bacillus Calmette-Guerin) and Corynebacterium parvum.

The present invention also relates to a method of immunizing an avian comprising administrating to the avian an effective amount of the vaccine according to the present invention. The method comprises administering to the animal an effective immunizing dose of the vaccine of the present invention. The vaccine may be administered by any of the methods well known to those skilled in the art, for example, by intramuscular, subcutaneous, intraperitoneal, intravenous, intranasally, orally, intradermal, iutrabursal, in ovo, or ocularly.

Preferred cysteine proteases are cysteine proteases from T. gallinae. These cysteine proteases from T. gallinae may either be prepared from T. gallinae or produced recombinantly (by other hosts). In the vaccine, also a mixture of different cysteine proteases may be administered, especially a mixture comprising at least two different cysteine proteases from T. gallinae. The proteases may be directly purified from T gallinae (as e.g. outlined in the example section) or produced recombinantly. In the example section, six proteolytic entities have been identified as six proteolytic spots. Provision of one or more of such spots in a vaccine is therefore a preferred embodiment of the present invention. Specifically preferred cysteine proteases are the proteases of T. gallinae. Specifically preferred proteases are: the T. gallinae protease comprising the amino acid sequence FSY-IADYPYTAR (Seq.ID.No.1) or FSYLADYPYTAR (Seq.ID.No.2); the T. gallinae protease comprising the amino acid sequence NSWGASWGEK (Seq.ID.No.3); the T. gallinae protease comprising the amino acid sequence NYWIVR (Seq.ID.No.4) or NYWLVR (Seq.ID.No.5); and the T. gallinae protease comprising the amino acid sequence VNVVEGDEADLATK (Seq.ID.No.6).

The vaccine according to the present invention is preferably administered i.m. or s.c; however, any other way for successfully eliciting an immune response to the cysteine proteases is suitable. The compositions according to the present invention may comprise a suitable veterinarily or pharmaceutically acceptable carrier, diluent, or excipient such as sterile water, physiological saline, glucose or the like. The compositions can also be lyophilized. The compositions can contain auxiliary substances, such as wetting or emulsifying agents, pH buffering agents, adjuvants, gelling or viscosity enhancing additives, preservatives, flavouring agents, colours, and the like, depending upon the route of administration and the preparation desired. Examples for such auxiliary substances include DMSO and/or tetradecyl maltoside (TDM). The most preferred route of administration of the compositions according to the present invention is oral administration, preferably via drinking water or via tablets.

The present invention is further described with the following examples and the drawing figures, yet without being limited thereto.

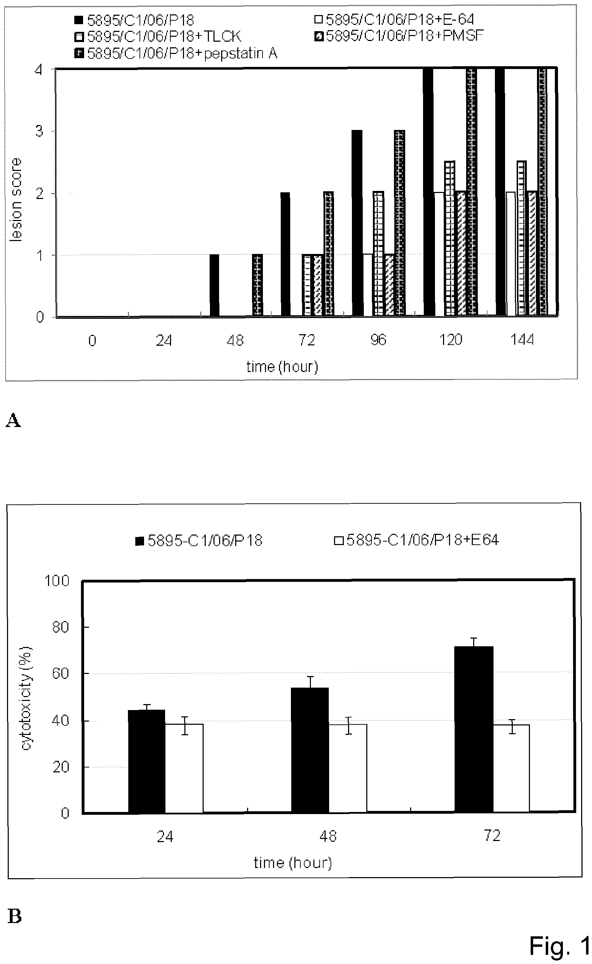

Fig. 1 shows the influence of peptidase inhibitors on the cytopathogenic effect of the cell-free filtrate. LMH monolayer was incubated with cell-free filtrate with and without different protease inhibitors. (A) Mean lesion scores, (B) cytotoxicity of LMH cells, as assessed by Promega CellTiter 96^{®} aqueous solution at different time points. Cell-free filtrate was obtained after 24 h of incubation of 10⁷ axenically grown protozoa cells obtained from T. gallinae clone 5895-Cl/06, passage18. Absorbance values for Promega CellTiter 96^{®} aqueous solution were recorded at 490 nm using ELISA reader.

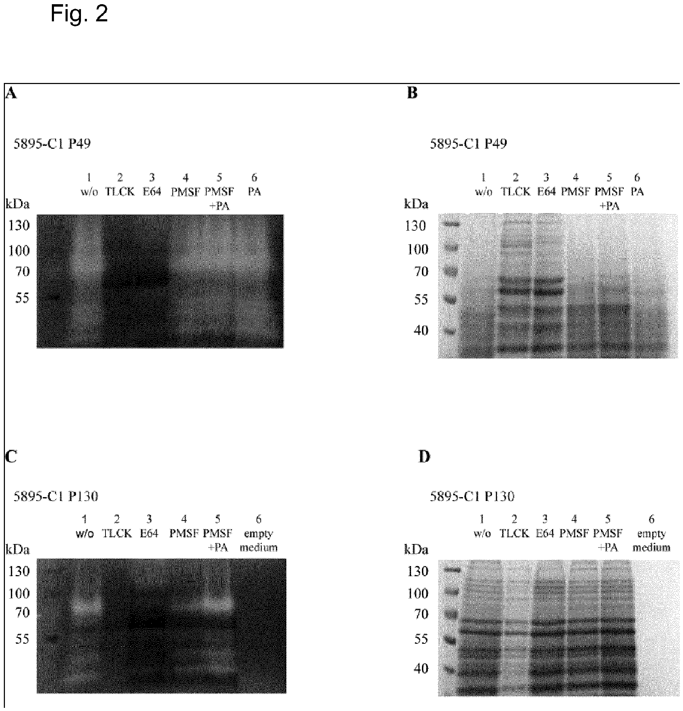

Fig. 2 shows the demonstration of the protease activity in cell-free filtrates from T. gallinae clone 5895-C1/06 by one-dimensional substrate SDS-PAGE. A) Substrate (0.2% gelatin) 1-D SDSPAGE (8%) with the low passage (49x) cell-free filtrate, B) conventional 1-D SDS PAGE (8%) with the low passage (49x) cell-free filtrate, C) Substrate (0.2% gelatin) 1-D SDS-PAGE (8%) with the high passage (130x) cell-free filtrate, and D) conventional 1-D SDS PAGE (8%) with the high passage (130x) cell-free filtrate.

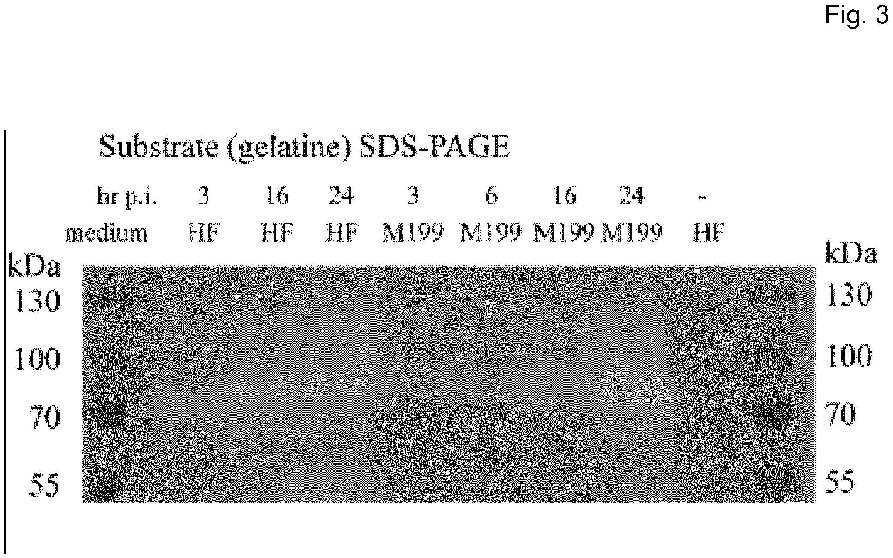

Fig. 3 shows the time-course experiment demonstrating the appearance of peptidases in the filtrate of the clone 5895-C1/06, P71 (107 T. gallinae cells) following incubation in the trichomonads growth medium (HF medium) and in the cell-culture medium (Medium 199). A) Substrate (0.2% gelatine) 1-D SDS-PAGE (8%).

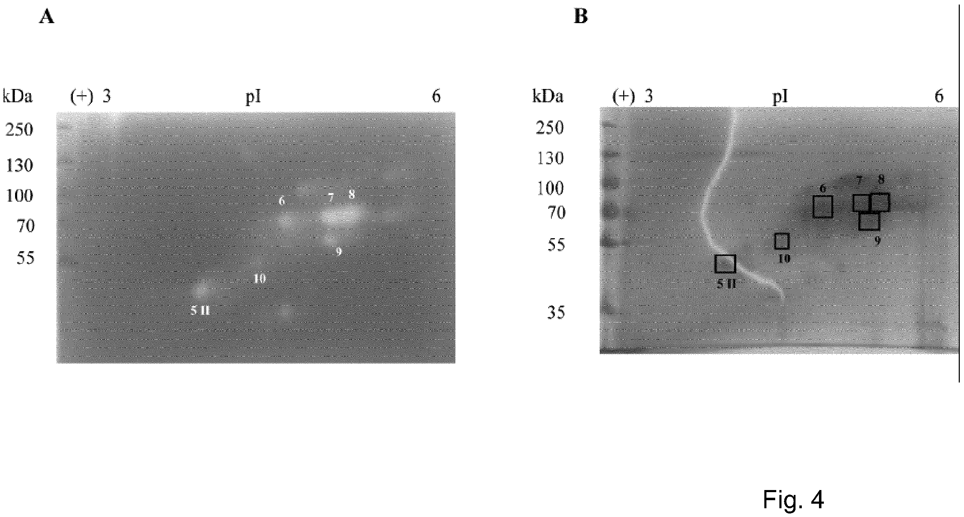

Fig. 4 shows the two-dimensional gel electrophoresis of the cell-free filtrate from clone 5895-C1/06, P53. A) Substrate (0.2% gelatine) SDS-PAGE (8%) as the second dimension and B) conventional SDS-PAGE (8%) as the second dimension.

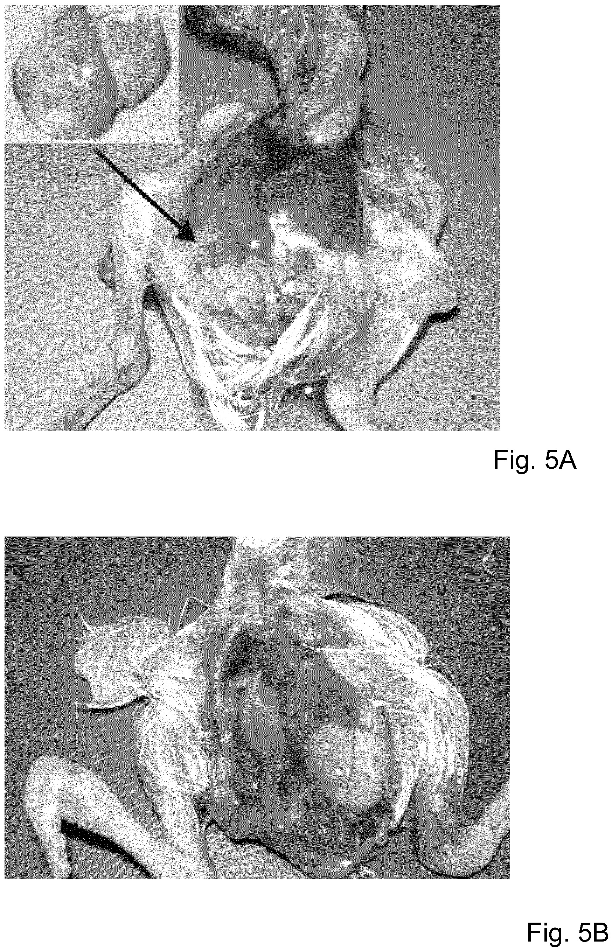

Fig. 5 shows the gross changes in the liver of spf chicken embryo infected with T. gallinae clone 5895-C1/06 P18, (A) yellow area in the liver (arrow) of embryo died at 7 dpi and (B) dilated gizzard of embryo killed at 10 dpi.

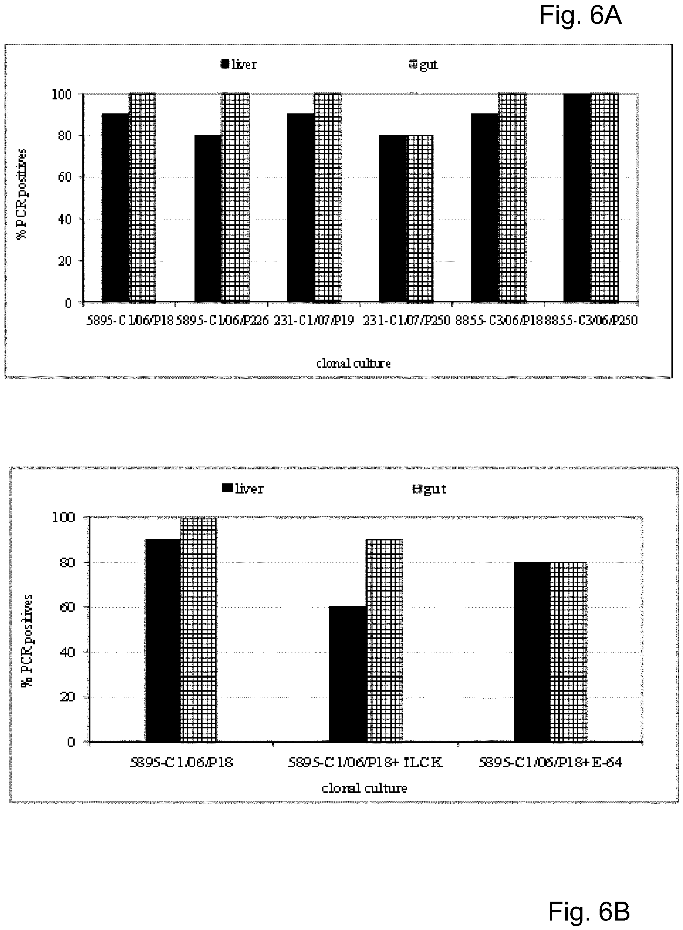

Fig. 6 shows percentage of positive PCR signals in the liver and gut (A) of embryos infected with different T. gallinae clonal cultures with varying in vitro passages low and high passage levels and (B) of embryos infected with T. gallinae clone 5895-C1/06 P18 either alone or coincubated with cysteine peptidase inhibitors (TLCK and E-64).

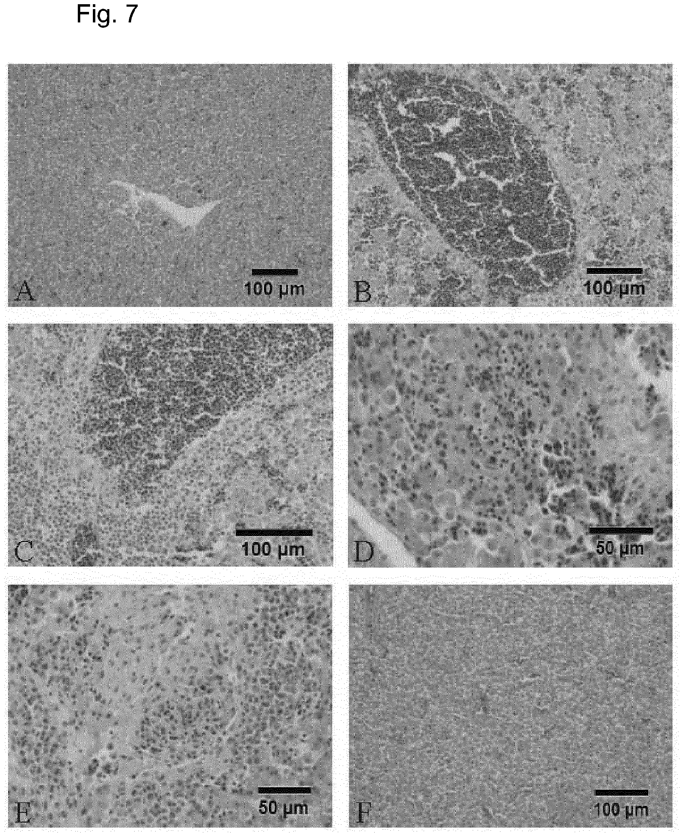

Fig. 7 shows histological sections of liver obtained from (A) negative control embryos, (b-e) spf chicken embryo infected with T. gallinae 5895-C1/06 P18 (B) congestion of hepatic blood vessels and sinusoids, (C) perivasular leukocytic cellular infiltration, (D) leukocytic cellular infiltration mainly lymphocytes in the hepatic parenchyma, (E) necrotic area with polymorphonuclear leukocytes and (F) liver from an embryo infected with 5895-C1/06 P18 in the presence of cysteine peptidase inhibitor (E-64). Haematoxylin and eosin staining.

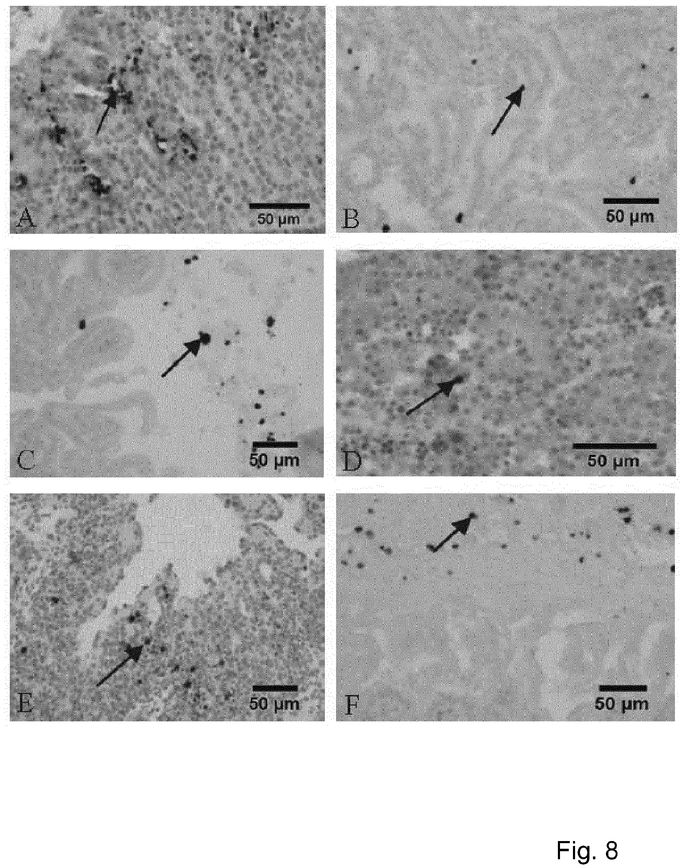

Fig. 8 shows detection of the nucleic acid of trichomonads by ISH in various organs of spf chicken embryos infected with T. gallinae 5895-C1/06 P18, such as (A) the gizzard, (B) the duodenum, (C) the caecum, (D) the liver, (E) the lung and (F) the gizzard from embryo infected with 5895-C1/06 P18 incubated with cysteine peptidase inhibitor (E-64).
Fig. 9 shows cathepsin L inhibitors.

### EXAMPLES

### 1. Cysteine peptidases, secreted by Trichomonas gallinae, are involved in the cytopathogenic effects on a permanent chicken liver cell culture

This example focused on the molecular characterization of cell-free filtrates from T. gallinae axenic cultures, which were previously shown to possess cytopathogenic effects on permanent chicken liver (LMH) cells. The identification of Clan CA, family C1, cathepsin L-like cysteine peptidases in the cell-free filtrate and demonstration of their involvement in the cytopathogenic effects of the filtrate show the virulent role of these peptidases in the pathogenesis of T. gallinae.

The present example demonstrated that the cytopathic effect of the cell-free filtrate on monolayers is reduced by the addition of the cysteine peptidase inhibitors TLCK and E-64. This showed that some of the important molecules involved in the monolayer destruction are cysteine peptidases. One-dimensional zymograms revealed no inhibitory effect of pepstatine A and PMSF on the proteolytic activity, demonstrating the inertness of these two inhibitors towards peptidases present in the filtrates. However, TLCK and E-64 efficiently inhibited the proteolytic activity of the filtrates. This generally correlated with results obtained with the cell-culture experiments. Furthermore, the results demonstrated that peptidases present in the filtrates, which were blocked by TLCK and E-64, were also partially responsible for the cytotoxicity of the filtrates.

The demonstration of Clan CA, family C1, cathepsin L like cysteine peptidases in the cell-free filtrate of T. gallinae by the 2-D substrate SDS-PAGE experiments combined with mass spectrometry supported the hypothesis that cysteine peptidases are the most abundant type of peptidases in the filtrates. Seven different proteolytic spots were detected by the 2-D substrate SDS-PAGE experiments showing the presence of at least 7 different peptidases in the cell-free extract of T. gallinae.

The present experiment had shown that the proteins secreted by T. gallinae possessed the proteolytic activity which contributed to the detachment of the monolayer. For the first time molecular characterization involving 1-D and 2-D zymograms in combination with mass spectrometric analysis showed that the cysteine peptidases are present in the pool of proteins secreted by T. gallinae. Finally, further demonstration that cysteine peptidases take part in the cytopathogenic effects of T. gallinae made these proteins excellent candidates for virulence factors.

### 1.1. Materials and Methods

### 1.1.1. Tested clonal culture

Cell-free filtrates of the T. gallinae axenic clonal culture named Trichomonas gallinae /Budgerigar/Austria/5895-C1/06 (shortly, clone 5895-C1/06), were investigated. The name assignment reflects the species of bird/country of origin/diagnostic number-clone number/year of isolation. In the present example a distinction between lower (P18, P49, P53) and high passages (P130) of the same clonal culture was made.

### 1.1.2. Cell culture

Permanent chicken liver (LMH) cells were grown in Medium 199 + Earle's salts + L-Glutamine (Invitrogen/Gibco, Paisley, U.K.). The medium was supplemented with 10% of FBS, 10% Tryptose Phosphate broth, penicillin (40,000 IU/ml) and streptomycin (40 mg/ml) (all Invitrogen/Gibco, Paisley, U.K). One milliliter of the media suspension containing 1x10⁶ cells was inoculated into 75 cm² flasks with filtered caps (Sarstedt, Wiener Neudorf, Austria) containing 6 ml medium. Cells were kept in a controlled atmosphere of 5% CO₂ at 37°C and around 85-90% humidity. After 72 h of incubation, a confluent monolayer of LMH cells was obtained with an average of 7x10⁶ cells per flask. Cells were passaged every three to four days depending on their density.

### 1.1.3. Preparation of cell-free filtrate from axenic T. gallinae culture

For the analysis on LMH monolayers cell-free filtrate was prepared in the following way: 10⁷ cells of a clonal culture of T. gallinae were incubated at 37°C for 24 h in the same medium as used for growth of LMH. Before incubation the protozoal cells were washed three times in PBS, (pH 7.2), trichomonad culture was centrifuged at 3300 x g for 5 min and then the supernatant from the culture was filtered through 0.22 µm cellulose acetate filters (Millipore, VWR).

For analysis in one- (1-D) and two-dimensional (2-D) zymogram and conventional SDS PAGE the filtrate was prepared in the following way: T. gallinae clonal culture was grown in Hollander fluid (HF) medium as described recently (Amin et al., Exp. Parasitol. 124 (2010), 202-208). After 24 h of incubation, the trichomonad culture was centrifuged at 3300 x g for 5 min; the pellet was washed three times in phosphate buffer saline (PBS, pH 7.2) and was then suspended in 1 ml HF medium without serum. Motile trichomonads were counted by a Neubauer cell counting chamber (Reichert, Buffalo, NY). The concentration of the inoculum was adjusted to 10⁷ motile trophozoites. 10⁷ trichomonad cells were grown for 3, 6, 16, 24 h in 10 ml HF medium without serum and the cell-free filtrate was prepared. The trichomonad culture was centrifuged at 3300 x g for 5 min, and then the supernatant from the culture was filtered through 0.22 µm cellulose acetate filters (Millipore, VWR). Afterwards, 1 mM PMSF, 5 µM of Pepstatin A, 1 mM TLCK and 270 µM E-64 were added to the cell-free filtrate and incubated for 30 min on ice before concentrating the proteins. Two types of filters were used to concentrate the proteins of the cell-free filtrate. Firstly, CEN-TRIPREP^{®} Centrifugal Filter Device 3K (Millipore, Vienna, Austria) was applied to reduce the initial volume of the filtrate (15ml) to 500 µl. After that, the samples were more concentrated using Amicon^{®} Ultra-0.5 3K centrifugal filter device (Millipore, Vienna, Austria) to obtain a final volume of 100 µl. Both types of filters were used following manufacturer's instructions. With the samples for 2-dimensional (2-D) sodium dodecyl sulphate (SDS)-polyacrylamide gel electrophoresis (PAGE) buffer was exchanged (three times) to 10 mM Tris-Cl pH 7.2 by using the same Amicon^{®} Ultra-0,5 3K centrifugal filter device (Millipore,Vienna, Austria). For all samples the protein concentration was measured by Bradford Reagent (Fermentas, Thermo Scientific).

### 1.1.4. Assessment of LMH monolayer

Each monolayer was investigated visually by an inverted 158 light microscope to detect the effect of the cell-free filtrates on LMH monolayer. Assesment of the monolayer was performed as recently described (Amin et al., Vet.Parasitol. (2011), doi: 10.1016/j.vetpar.2011.11.037).

### 1.1.5. Effect of peptidase inhibitors on the cell-free filtrate

Firstly, preliminary experiments were done to establish the concentration of each peptidase inhibitor which was not toxic to the monolayer cell culture. In these initial experiments different concentrations of the following peptidase inhibitors were added to not-infected monolayer: 0.2 mM, 0.5 mM, 1 mM Phenylmethylsulfonyl fluoride (PMSF) as serine peptidase inhibitor, 1 µM, 2.5 µM, 5 µM pepstatin A as aspartic peptidase inhibitor, 135 µM, 540 µM, 1 mM N-α-p-Tosyl-L-Lysine chloromethyl ketone (TLCK) as cysteine and some serine peptidase inhibitor, and 70 µM 140 µM, 270 µM L-3-carboxyl-2,3-trans-epoxysuccinylleucylamido (4-guanidino) butane (E-64) as cysteine peptidase inhibitor (all purchased from Sigma-Aldrich, Steinheim, Germany).

### 1.1.6. Cytotoxicity assay

In order to investigate the cytotoxicity of cell-free filtrate on cell culture, CellTiter 96^{®} aqueous one solution cell proliferation assay (Promega Corporation, Madison, USA) was used following manufacturer's instructions. The cell-free filtrate from 10⁷ trichomonad cells was prepared as described above and 270 µM of E-64 as cysteine peptidase inhibitor was added to cell-free filtrate before its incubation with the monolayer. Cytotoxicity assay was performed as described recently by Amin et al. (2011).

### 1.1.7. One- and two dimensional gels electrophoresis and zymographic analysis

For one dimension (1-D) zymographic analysis, 24 µg of concentrated p 183 proteins from cell free filtrates with and without peptidase inhibitors were separated by discontinuous SDS-PAGE (8%) copolymerized with 0.2% gelatin as substrate. In parallel, the same samples were investigated by conventional 1-D SDS-PAGE (8%). After electrophoresis, the substrate gel was incubated in 2.5% Triton X-100 (Bio-Rad Laboratories GmbH, Vienna, Austria) for 1 h at room temperature to remove SDS. Subsequently the peptidases were activated by incubating the gel in zymogram development buffer (Bio-Rad Laboratories GmbH, Vienna, Austria) for 24 h at 37 °C. All gels were stained with PageBlue™ Protein Staining Solution (Fermentas, Fisher Scientific Österreich, Vienna, Austria) and clear bands in gelatin gels were indicative of proteolytic activity. All experiments were repeated twice.

For 2-D zymographic analysis of the concentrated proteins from cell-free filtrate buffer was exchanged to rehydration buffer (8M Urea (GE Healthcare, UK), 2% CHAPS (Sigma-Aldrich, Steinheim, Germany), 50 mM DTT (Sigma-Aldrich, Steinheim, Germany), 0,2% Servalyt^{®}-Ampholytes pH 3-5 (Serva), trace bromphenol blue (Sigma-Aldrich, Steinheim, Germany) via 2eba™ Spin Desalting Columns, 7K MWCO (Pierce, Fisher Scientific Österreich, Vienna, Austria) according to manufacturer's instructions. For first-dimension of both silver stained and PageBlue™ stained zymogram gels, 240 µg protein was applied to IPG-strip (7 cm, pH 3-6, Bio-Rad Laboratories GmbH, Vienna, Austria) by passive in-gel rehydration (16 h at room temperature). Isoelectric focusing (IEF) was carried out in a Protean IEF cell (Bio-Rad Laboratories GmbH, Vienna, Austria): voltage was increased in the slow ramp mode; 100 V in 1 h, 250 V in 1 h, 500 V in 1 hour, 1000 V in 1.5 h, 4000V in 1 h followed by focusing at 4000 V until 18 000 Vhs were reached. For both types of analyses, the IPG strips were equilibrated before the second dimensional run first in 6M Urea (GE Healthcare, UK), 2% SDS (Merck, VWR), 0.375M Tris-Cl (Merck, VWR) 20% glycerol (Sigma-Aldrich, Steinheim, Germany) pH 8.8 containing 2% DTT (Sigma- Aldrich, Steinheim, Germany) and afterwards in the same buffer supplemented with 2.5% iodoacetamide (Sigma-Aldrich) for 10 min at room temperature each. In the second-dimension proteins were resolved by SDS-PAGE (8%) and substrate (0.2% 209 gelatin) SDS-PAGE (8%) for silver-stained gels and zymograms, respectively. Silver-staining was performed according to Shevchenko et al. (Anal. Chem. 68 (1996), 850-858. Zymogram gels were treated and stained as described for 1-D zymograms gels. Clear spots were indicative for proteolytic activity. Images of all gels (1-D and 2-D) were taken with Molecular Imager ChemiDoc™ XRS System (Bio-Rad Laboratories GmbH, Vienna, Austria) using Quantity One v4.6.3 software (Bio-Rad Laboratories GmbH, Vienna, Austria).

### 1.1.8. Protein digestion, peptide extraction and mass spectrometric analysis

Selected spots from 2-D gels were excised, washed, destained, reduced with DTT (Sigma-Aldrich, Steinheim, Germany) and alkylated with Iodoacetamide (Sigma- Aldrich, Steinheim, Germany). The in-gel digest was carried out with trypsin (Trypsin Gold, Mass Spectrometry Grade, Promega Corporation, Madison, USA) (Shevchenko et al., 1996) and after extraction the dried peptides were de-salted using µZip-Tips C18 (Millipore, VWR, Vienna, Austria) according to the manufacturer's instructions. De-salted peptides (0.5 µl) were spotted onto a pre-spotted Anchor-Chip MALDI (PAC target, Bruker Daltonik GmbH, Leipzig, Germany).

Data were acquired on a Matrix Assisted Laser Desorption Ionisation Tandem Time-of-Flight (MALDI-TOF/TOF) mass spectrometer (Ultraflex II, Bruker Daltonik GmbH, Leipzig, Germany) in MS and MS/MS modes. Spectra processing and peak annotation were carried out using FlexAnalysis and Biotools (Bruker Daltonik GmbH, Leipzig, Germany).

### 1.1.9. Data analysis and de novo peptide sequencing

Processed spectra were searched via Mascot in the Swiss-Prot database (release 56.5) or in NCBInr (20111005) using the following search parameters: taxonomy trichomonas; global modifications carbamido-methylation on cysteine; variable modifications oxidation on methionine; MS tolerance 100 ppm; MS/MS tolerance 1 Da; one missed cleavage allowed. Identifications were considered statistically significant where p < 0.05.

Peptide de Novo sequencing was carried out manually using FlexAnalysis. These sequences were then used for a homology search using MS Homology in ProteinProspector 5.9.0. http://prospector.ucsf.edu/prospector/cgibin/msform.cgi?form=mshomology. The searched database was Uni-ProtKB.2011.01.11 with following parameters: taxonomy search in Trichomonas vaginalis, 0.5k Da mass tolerance, no enzyme digest, one missed cleavage allowed, minimal match of peptides 1 and BLOSUM62 as score matrix.

### 1.2. Results

### 1.2.1. Influence of peptidase inhibitors on the cytopathic effect of cell-free filtrate from axenically grown T. gallinae

In order to establish the maximal concentration of each peptidase inhibitor without toxicity for the monolayer, incubations with different concentrations were performed. Accordingly, the suitable concentration for each inhibitor was used in this study. The results showed that the addition of either 1 mM PMSF, 270 µM E64 or 135 µM TLCK to the cell-free filtrate of T. gallinae partially inhibited cytopathic effects induced by trichomonads (Fig. 1). The inhibition was assayed by the detachment of the monolayer in comparison to the filtrate without inhibitors (Fig. 1A). After applying peptidase inhibitors the monolayer destruction was reduced, but differences between the actions of these inhibitors were noticed. The E-64, cysteine peptidase inhibitor, produced the best inhibition causing the lowest destruction of the monolayer. The application of Pepstatin A (5 µM), aspartic peptidase inhibitor, had no inhibitory effect on the filtrate (Fig. 1A).

The effect of the E-64, cysteine peptidase inhibitor, on the cell-free filtrate was also assayed by CellTiter 96^{®} aqueous one solution cell proliferation assay and showed the significant reduction in cytotoxicity (Fig. 1B). After 72 h of incubation the maximum cytotoxicity of T. gallinae clone 5895-C1/06, passage 18, on LMH cells, produced by the cell-free filtrate without E-64, was 70.9%. In the presence of the inhibitor the toxicity of the filtrate could be reduced to 37.1%.

### 1.2.2. Demonstration of peptidase activity in T. gallinae cell-free filtrates using one-dimensional substrate gel electrophoresis

In order to detect whether T. gallinae cells secreted peptidases into trichomonad-growth medium (HF medium), concentrated cell-free filtrates from clone 5895-C1/06 with and without peptidase inhibitors were separated by SDS-PAGE with gelatin copolymerized as substrate (Fig. 2). Additionally, filtrates of lower (P49) and high passages (P130) from clone 5895-C1/06 were compared. The zymogram of the cell-free filtrate from P49 of clone 5895-C1/06 without peptidase inhibitors showed a proteolytic region of at least five different clear bands with molecular weights of approximately 38, 41, 50, 80 and 110 kDa (Fig. 2A). In contrary, the zymogram analysis of the cell-free filtrates from P130 of clone 5895-C1/06 demonstrated weaker proteolytic activity (Fig. 2C). The clear bands indicative for proteolytic activity were of weaker intensity and the 110 kDa band present in zymogram of cell-free filtrate from P49 was absent. No proteolytic bands were observed on both zymograms of cell-free filtrates containing peptidase inhibitors TLCK (cysteine and some serine peptidases) and E-64 (cysteine peptidases) (Fig. 2A, C). Samples treated with PMSF (serine peptidase inhibitor) and pepstatin A demonstrated the same proteolytic regions as the sample without inhibitors (Fig. 2A, C). In parallel, the same samples were investigated by conventional SDS-PAGE (figure 2B and 2D). Cell-free filtrate from clone 5895-C1/06 P49 containing TLCK and E-64 produced a pattern of sharp bands of different molecular weights (Fig 2B). In contrary, in samples without inhibitors or those with PMSF and pepstatin A, this pattern was not prominent and the remaining bands were not sharp. This additionally confirmed the presence of peptidase activity in the cell-free filtrate of P49 of clone 5895-C1/06 which could be specifically blocked by addition of TLCK and E-64. Interestingly, conventional SDS-PAGE of filtrates from P130 of clone 5895-C1/06 demonstrated sharp bands in all samples even in the ones without peptidase inhibitors (Fig. 2D).

In the time-course experiment in which passage 71 of clone 5895-C1/06 was incubated in trichomonad growth medium (HF medium) and cell-culture medium (Medium 199), peptidases were already demonstrated in the filtrate collected after 3 h of incubation of 10⁷ trichomonad cells (Fig. 3). The intensity of the proteolytic activity in cell-free filtrates increased with the duration of incubation.

### 1.2.3. Detection of Clan CA, family C1, cathepsin L-like cysteine peptidases in T. gallinae cell-free filtrate by MS analysis of peptidase spots in 2-D gel

In order to further characterize the proteolytic activity of the cell-free filtrate from T. gallinae, the concentrated proteins of cell free-filtrates from passage 53 of clone 5895-C1/06 were investigated by conventional and substrate 2-D gel electrophoresis (Fig. 4). Zymographic analysis identified at least seven spots with proteolytic activity between 35 and 100 kDa, which displayed pI values between 3.7 and 5.3 (calculated according to linear distribution of pH across the strip). The MS analysis of six spots of conventional 2-D gels, which corresponded to the proteolytic spots found in 2-D zymograms, identified the presence of Clan CA, family C1 and cathepsin L-like cysteine peptidases in cell-free filtrate of T. gallinae. In five proteolytic spots (6, 7, 8, 9, 10) a peptide with the same mass (m/z 1466.6) was identified. Manual peptide de Novo sequencing of this charged peptide identified the following sequence: FSY[I/L]ADYPYTAR. The MS homology search matched this peptide to different Clan CA, family C1, cathepsin L-like cysteine peptidases of T. vaginal is. In spot 8 (70 kDa, pI 5.2) two additional peptides with masses of m/z 850 and m/z 1121 could be identified. Manual sequencing of the tandem mass spectra corresponding to the single peptides identified following peptide sequences: NYW[I/L]VR (m/z 850.5) and NSWGASWGEK (m/z 1121.4). The MS homology search using all three peptides matched these peptides with highest homology to T. vaginalis Clan CA, family C1, cathepsin L-like cysteine peptidase TVAG_355480 (accession number XP_001310117).

The MS/MS spectrum of the sixth proteolytic spot, protein s 312 pot 5II (45 kDa, pI 3.7), identified two peptides with masses of m/z 850.5 and m/z 1879.8. The manual peptide de Novo sequencing identified peptides with the sequences: NYWI/LVR (m/z 850) and VNVVEGDEADLATK (m/z 1879). The MS homology search using either combination of NYWI/LVR and VNVVEGDEADLATK matched these peptides with the highest homology to T. vaginalis Clan CA, family C1, cathepsin L-like cysteine peptidase TVAG_298080 (accession number XP 001316414) and T. vaginalis CP39-Cytotoxic cysteine proteinase (accession number ABX56032).

### 2. Peptidase inhibitors are able to neutralize the virulence of Trichomonas gallinae in vivo

In this example a suitable in vivo system capable to assess the pathogenicity of T. gallinae was established in order to investigate the pathogenicity of different clonal cultures of T. gallinae in spf chicken embryos. PCR and ISH were carried out to screen the spread of trichomonads in liver and gut of embryos infected with different clonal cultures. Both techniques were found to be effective tools for demonstration of trichomonads in these organs. Most severe histological changes were detected in the liver. This may be due to the short incubation period, as the infection continued for ten days only, at which the lesions in other organs might still have been in the early stage. In addition to the oropharynx, the liver is a second predilection site for virulent T. gallinae strains in vivo. Liver lesions range from the presence of a few, yellow necrotic areas to a nearly complete caseation of the organ. In agreement with this, yellow necrotic areas on the liver surface of some embryos were noticed in the present example.

Previous reports mentioned that T. gallinae is mainly inhabiting the upper part of the digestive tract and findings in the lower parts of the proventriculus is uncommon. Contrary, in the present example, the protozoal cells were demonstrated by ISH in the gizzard and intestine of some infected embryos with absence of any lesions. This could be due to the applied in vivo system, using spf chicken embryos. The presence of protozoa in the gut of the embryos could be explained by the oral uptake of the flagellates together with the nutrients, whereas generalization and spread into other organs occurred at a second stage, following penetration of the gut epithelium and entrance into the bloodstream.

In the present experiment spf chicken embryos were established successfully as a simple model to evaluate the virulence of different clones of T. gallinae in vivo. For the first time it could be demonstrated that the progression of avian trichomonosis is linked with the presence of cysteine peptidases which can be neutralized by co-application of peptidase inhibitors without toxicity to the host. Such peptidase inhibitors had no direct cytotoxic effect on the viability of the trichomonads. This shows that the reduction of pathogenicity of T. gallinae in the presence of peptidase inhibitors is due to the inhibition of cysteine peptidase activity rather than a direct effect on the trichomonad cells. These findings underline the importance of cysteine peptidases as drug targets for chemotherapy.

### 2.1. Materials and Methods

### 2.1.1. Clonal cultures

Three genetically different clonal cultures of T. gallinae named Trichomonas gallinae/Budgerigar/Austria/5895-C1/06 (briefly, clone 5895-C1/06) passaged 18 (P18) and 226 (P226) times, Trichomonas gallinae/Racing Pigeon/Austria/8855-C3/06 (clone 8855-C3/06) passaged 18 (P18) and 250 (P250) times, and Trichomonas gallinae/Racing Pigeon/Austria/231-C1/07 (clone 231-C1/07) passaged 19 (P19) and 250 (P250) times were used in this experiment. The assignment reflects the species of bird/country of origin/diagnostic number-clone number/year of isolation.

### 2.1.2. Propagation of T. gallinae

The axenic clonal cultures of T. gallinae were grown in Hollander fluid (HF) medium, as described above (Amin et al., 2010). At first, clonal cultures which underwent only a few passages were investigated. Higher passages were obtained by transferring 1 ml of the respective culture into 9 ml of fresh HF medium every 48 h. Assessing the virulence as well as the effect of peptidase inhibitors was performed with an equal passage number (P18). After 24 h of incubation, the cultures were centrifuged at 3300 x g for 5 min (Hettich Rotanta 460, Tuttlingen, Germany). The pellet was washed three times by addition of phosphate buffer saline (PBS, pH 7.2) followed by centrifugation. For counting cells were suspended in 1 ml PBS buffer and motile trichomonads were determined by a Neubauer cell counting chamber (Reichert, Buffalo, NY, USA). The concentration of the inoculum was adjusted to contain approximately 5x10⁶ actively motile trophozoites suspended in 200 µl PBS buffer. The viability of trichomonad cells was evaluated using trypan blue staining.

### 2.1.3. Investigated peptidase inhibitors

In order to determine the suitable concentrations of cysteine peptidase inhibitors that have no toxic effect on protozoal cells, different concentrations (1mM, 800 µM and 675 µM) of N-α-130 p-Tosyl-L-Lysine chloromethyl ketone (TLCK) and (270 µM, 140 µM and 70 µM) of L-3-carboxyl-2,3-trans-epoxysuccinylleucylamido (4-guanidino) butane (E-64) (all purchased from Sigma-Aldrich, Steinheim, Germany) were added to the axenic trichomonad cultures. The concentration that maintains the viability of T. gallinae trophozoites as revealed by trypan blue staining was used in this experiment.

### 2.1.4. Embryo inoculation and examination

For each treatment, ten 9-day old embryonated spf chicken eggs were inoculated with 200 µl of the individual sample of protozoa suspended in sterile PBS via the allantoic cavity following a standard egg inoculation procedure (Senne, 1998). In addition, ten eggs were inoculated in the same way with sterile PBS with and without an adjusted concentration of peptidase inhibitors, to be used as a control. The inoculated eggs were sealed with melted paraffin and incubated at 37°C at a relative humidity of 55-60%. The survival of the embryos was monitored by candling the eggs daily. At the end of the incubation period (10 days) the eggs were placed at -20°C for two hours to kill the embryos and to avoid hatching. The allantoic fluid was harvested and examined microscopically for the detection of flagellates, before the embryos were necropsied. Afterwards, samples from different organs (liver, heart, lung, proventriculus, gizzard, kidney, spleen, and intestine) were preserved in 10% buffered neutral formalin and later on embedded in paraffin wax for histological examination. To demonstrate microscopic lesions in the tissues haematoxylin and eosin stain was performed. For specific 150 demonstration of the protozoa or its nucleic acid in the tissue sections in situ hybridization (ISH) was applied following the protocol of Liebhart et al. (J. Comp. Pathol. 135 (2006), 237-242). Additionally, samples from liver and gut were preserved at -20°C for polymerase chain reaction (PCR). DNA was extracted from tissue samples applying the Dneasy® Tissue Kit (Qiagen, Vienna, Austria), according to the manufacturer instructions. PCR was performed following the protocol of Grabensteiner and Hess (Vet. Parasitol. 142 (2006), 223-230). The embryos that died during the experiments were exposed to the same examination procedures. During necropsy autoclaved scissors and disposable tweezers were used for collecting the samples to avoid cross contamination.

### 2.2. Results

### 2.2.1. Necropsy of embryos infected with different clonal cultures of T. gallinae

Examination of the embryos infected with T. gallinae clonal culture 5895-C1/06 P18 revealed gross changes either in the embryos which died at 7, 8 and 9 dpi or in those embryos which were killed at the end of the incubation period (10 dpi) (Figure 5): An enlarged, severely congested liver was observed in six out of ten infected embryos. Of these six embryos only two had yellowish areas or tiny creamy-white foci, especially at the periphery of the hepatic lobule. In these embryos congested kidneys were observed. Additionally, dilated gizzards were seen in some embryos of this group. However, no lesions were observed in the heart, lung, proventriculus, spleen and intestine.

In contrast, no mortalities were recorded in the group of embryos infected with T. gallinae clone 8855-C3/06 P18. After killing of the embryos at 10 dpi congested and enlarged livers were observed in four embryos. In one embryo the kidney was congested. Except one embryo which died on 9th day no mortalities were observed in the group inoculated with clone 231-C1/07 P19. The gross examination revealed a congested and slightly enlarged liver, a finding also present in one embryo killed at the end of the experiment. Live trichomonads were present in the allantoic fluid investigated from those embryos which died following infection. No macroscopic lesions were observed at 10 dpi in any of the embryos inoculated with different clonal cultures of T. gallinae after long term propagation, similar to the negative control group.

### 2.2.2. Gross examination of embryos infected with a virulent T. gallinae clone coincubated with peptidase inhibitor

No mortalities were recorded in the group of embryos infected with T. gallinae clone 5895-C1/06 P18 coincubated with both types of peptidase inhibitors. After killing of the embryos at 10 dpi no gross lesions in the investigated internal organs (liver, heart, lung, proventriculus, gizzard, kidney, spleen, and intestine) were observed in any of these embryos. Moreover, no mortalities or macroscopical changes were demonstrated in embryos inoculated with PBS alone or in the presence of the adjusted amount of peptidase inhibitors.

### 2.2.3. Polymerase chain reaction

Positive PCR signals were demonstrated in the liver and gut of most infected embryos with different T. gallinae clones at both low and high passage numbers (Figure 6a). No differences were noticed regardless of the passage numbers between samples processed from different clonal cultures. Moreover, positive PCR results were obtained from the liver and gut of embryos infected with clone 5895-C1/06 P18 and coincubated with TLCK or E-64 (Figure 6b). According to the obtained PCR results, there were no considerable differences comparing the infection either in the presence or absence of E-64. Additionally, TLCK had obviously a more distinct inhibitory effect on the infiltration of the flagellate to the liver than E-64.

### 2.2.4. Histopathology

Apparent differences were noticed between liver samples taken from control embryos (Figure 7a) and those from infected groups. The microscopical examination of livers from embryos infected with T. gallinae clone 5895-C1/06 P18 and stained by haematoxylin and eosin staining revealed severe congestion and dilatation of hepatic blood vessels and blood sinusoids (Figure 7b). Moreover, multiple haemorrhagic areas were scattered all over the hepatic parenchyma. Perivascular leukocytic cellular infiltration, mainly heterophils and lymphocytes, were seen (Figure 7c). Areas of leukocytic cellular infiltration, consisting mainly of mononuclear cells, were observed in the hepatic parenchyma (Figure 7d). Additionally, a caseated necrotic mass was noticed in the hepatic parenchyma with leukocytic cellular infiltration mainly heterophils, especially at the periphery of the lesion (Figure 7e). Most of these histological lesions were present in six of the ten embryos infected with the above mentioned clone. In addition, congestion of the renal blood vessels was observed in two embryos within this group.

Mild to severe congestion of the hepatic blood vessels and blood sinusoids were detected in the liver of four out of ten embryos infected with the clonal culture 8855-C3/06 P18, while among the embryos infected with 231-C1/07 P19 mild congestion of the hepatic blood vessels was observed in only two embryos.

Following haematoxylin and eosin stain, no specific colour reaction of trichomonads or their organelles could be achieved. In contrast, positive signals in histological tissue sections by ISH were clearly noticed as dark blue stained cells. Protozoal cells were demonstrated adhered to the cuticle of the gizzard of embryos infected with different clonal cultures regardless of the passage number. However, for embryos infected with clone 5895-C1/06 or 8855-C3/06, both P18 the protozoa were also attached to the mucosal epithelium of the gizzard (Figure 8a). Moreover, trichomonad cells were also observed in the gastric muscular layer and in the submucosal glands of the proventriculus of these embryos. Furthermore, flagellates were noticed either in the intestinal lumen or attached to the intestinal villi in the duodenum and caecum (Figure 8b and c), in the hepatic parenchyma (Figure 8d) and in the lung where protozoon were scattered in the pulmonary tissues and adhered to the epithelium of the atria wall (Figure 8e). Although, the parasites were detected by ISH in the proventriculus, gizzard, lung, and intestine of some embryos infected with 5895-C1/06 and 8855-C3/06 both P18, no microscopical lesions were noticed in these organs. Moreover, the nucleic acid of the flagellates was demonstrated in the splenic capsule and in the renal tissue of one embryo infected with clone 8855-C3/06 P18. Overall, the microscopical examination of the heart revealed no pathological changes and no trichomonad cells were present in this organ by ISH. Positive signals were seen in the blood vessels of different organs with parasites emigrating into the tissues leading to extravascular location.

In general, no histological changes were observed in all examined organs namely liver, lung, heart, spleen, kidney, proventriculus, gizzard and intestine obtained from embryos infected with different clonal cultures which underwent a high number of in vitro passages. Tissues processed from the control group were found normal and no trichomonads could be detected by ISH.

### 2.2.5. Effect of peptidase inhibitors on the pathogenicity of T. gallinae

The optimum concentration that had no effect on the viability of T. gallinae trophozoites as revealed by trypan blue staining was used in this experiment. These concentrations were 675 µM for TLCK and 270 µM for E-64, respectively. Consequently, no cytotoxic effect on the embryos, no mortalities or pathomorphological changes in the investigated organs were recorded in the negative control groups. The histopathological examination of different organs of the embryos infected with T. gallinae clone 5895-C1/06 P18 in the presence of peptidase inhibitors (TLCK and E-64) revealed no histological changes as demonstrated exemplarily for one of the liver samples (Figure 7f). However, the nucleic acid of trichomonds was demonstrated by ISH in the hepatic parenchyma and in some of these embryos trophozoites were noticed attached to the cuticle of the gizzard (Figure 8f).

Preferred embodiments according to the present invention:
1. Protease inhibitors for use in the treatment of a Trichomonas gallinae infection of an animal, selected from the orders Galliformes, Passeriformes, Psittaciformes, Columbiformes and Falconiformes.
2. Protease inhibitors according to embodiment 1 wherein the protease inhibitors are selected from cysteine protease inhibitors and serine protease inhibitors, especially cysteine protease inhibitors.
3. Protease inhibitors according to embodiment 1 or 2, wherein the protease inhibitors are cysteine protease inhibitors selected from the group consisting of E-64 (trans-Epoxysuccinyl-L-leucylamido(4-guanidino)butane, L-trans-3-Carboxyoxiran-2-carbonyl-L-leucylagmatine, N-(trans-Epoxysuccinyl)-L-leucine 4-guanidinobutylamide), TLCK (tosyl-L-lysine chloromethyl ketone), TPCK (tosyl-L-phenylalanine chloromethyl ketone), or mixtures thereof.
4. Protease inhibitors according to any one of embodiments 1 to 3 for use in the treatment of a Trichomonas gallinae infection of an animal, selected from the species Columba livia, Melopsittacus undulates, Serinus canaria forma domestica, or Falco spp..
5. Protease inhibitors according to any one of embodiments 1 to 4, wherein the inhibitors are administered orally to the animal, preferably wherein oral administration includes administration of a solution of cysteine protease inhibitors or cysteine protease inhibitors in tablet form.
6. Method for treating a Trichomonas gallinae infection of an animal, selected from the orders Galliformes, Passeriformes, Psittaciformes, Columbiformes and Falconiformes, wherein an effective amount of a protease inhibitor, especially a cysteine protease inhibitor, is administered to an animal.
7. Use of a protease inhibitor, especially a cysteine protease inhibitor for the manufacture of a medicament for the treatment of a Trichomonas gallinae infection of an animal, selected from the orders Galliformes, Passeriformes, Psittaciformes, Columbiformes and Falconiformes.
8. Vaccine against a Trichomonas gallinae infection of an animal, selected from the orders Galliformes, Passeriformes, Psittaciformes, Columbiformes and Falconiformes, comprising a cysteine protease and an adjuvant.
9. Vaccine against a Trichomonas gallinae infection of an animal, selected from the orders Galliformes, Passeriformes, Psittaciformes, Columbiformes and Falconiformes, comprising a nucleic acid molecule, especially a DNA molecule, encoding a cysteine protease and an adjuvant.

## Claims

1. Cysteine protease inhibitors for use in the oral treatment of a Trichomonas gallinae infection of an animal, selected from the orders Galliformes, Passeriformes, Psittaciformes, Columbiformes and Falconiformes.

2. Cysteine protease inhibitors according to claim 1, wherein the protease inhibitors are cysteine protease inhibitors selected from the group consisting of E-64 (trans-Epoxysuccinyl-L-leucylamido(4-guanidino)butane, L-trans-3-Carboxyoxiran-2-carbonyl-L-leucylagmatine, N-(trans-Epoxysuccinyl)-L-leucine 4-guanidinobutylamide), TLCK (tosyl-L-lysine chloromethyl ketone), TPCK (tosyl-L-phenylalanine chloromethyl ketone), or mixtures thereof.

3. Cysteine protease inhibitors according to claim 1 or 2 for use in the oral treatment of a Trichomonas gallinae infection of an animal, selected from the species Columba livia, Melopsittacus undulates, Serinus canaria forma domestica, or Falco spp..

4. Cysteine protease inhibitors according to any one of claims 1 to 3 for use in the oral treatment of a Trichomonas gallinae infection of an animal, wherein oral treatment includes administration of a solution of cysteine protease inhibitors or cysteine protease inhibitors in tablet form.

5. Method for treating a Trichomonas gallinae infection of an animal, selected from the orders Galliformes, Passeriformes, Psittaciformes, Columbiformes and Falconiformes, wherein an effective amount of a cysteine protease inhibitor is administered orally to an animal.

6. Use of a cysteine protease inhibitor for the manufacture of a medicament for the oral treatment of a Trichomonas gallinae infection of an animal, selected from the orders Galliformes, Passeriformes, Psittaciformes, Columbiformes and Falconiformes.

7. Vaccine against a Trichomonas gallinae infection of an animal, selected from the orders Galliformes, Passeriformes, Psittaciformes, Columbiformes and Falconiformes, comprising a cysteine protease and an adjuvant.

8. Vaccine against a Trichomonas gallinae infection of an animal, selected from the orders Galliformes, Passeriformes, Psittaciformes, Columbiformes and Falconiformes, comprising a nucleic acid molecule, especially a DNA molecule, encoding a cysteine protease and an adjuvant.
